(19) Europäisches Patentamt
European Patent Office
Office européen des brevets

(11) **EP 3 406 867 A1**

(12) **EUROPEAN PATENT APPLICATION**

(43) Date of publication:
**28.11.2018 Bulletin 2018/48**

(51) Int Cl.:
*F01M 11/10* (2006.01)  *F02C 9/00* (2006.01)

(21) Application number: **18173490.6**

(22) Date of filing: **22.05.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.05.2017  US 201715602142**

(71) Applicant: **General Electric Company**
**Schenectady, NY 12345 (US)**

(72) Inventors:
• **ZHANG, Hua**
**Greenville, SC 29615 (US)**
• **CHAPMAN, Seth Harmon**
**Greenville, SC 29615 (US)**
• **O'DONNELL, Keegan Saunders**
**Greenville, SC 29615 (US)**
• **STOVER, Jan Terry**
**Greenville, SC 29615 (US)**

(74) Representative: **Lee, Brenda**
**GE International Inc.**
**Global Patent Operation - Europe**
**The Ark**
**201 Talgarth Road**
**Hammersmith**
**London W6 8BJ (GB)**

(54) **TURBOMACHINE LUBRICATING OIL ANALYZER SYSTEM, COMPUTER PROGRAM PRODUCT AND RELATED METHODS**

(57)    Various embodiments of the invention include a system 102 having: at least one computing device 124 at least one computing device 124 configured to monitor a lubrication oil 300 by performing actions including: determining an initial ideal remaining life for the lubrication oil 300; determining a temperature-based remaining life for the lubrication oil 300 based upon a temperature measurement of the lubrication oil 300; calculating a contamination factor of the lubrication oil 300 based upon a non-particle count sample of the lubrication oil 300; determining an updated ideal life remaining for the lubrication oil 300 based upon the contamination factor, the initial ideal remaining life, and the temperature-based remaining life; and determining an actual life remaining for the lubrication oil 300 based upon the updated ideal life remaining and an actual life lost for the lubrication oil 300.

FIG. 3

**Description**

FIELD OF THE INVENTION

The subject matter disclosed herein relates to turbomachine systems. More particularly, the subject matter disclosed herein relates to analyzing lubrication oil in turbomachine systems, for example, gas turbomachines or steam turbomachines.

BACKGROUND OF THE INVENTION

Turbomachines, for example, gas turbines and/or steam turbines, use lubricating oil to reduce the frictional coefficient between machine components. While many turbomachines are delivered and installed by a manufacturing and/or selling entity, these turbomachines are frequently managed (over their lifetime) by the customer that purchases the turbomachine. In order to ensure that the lubricating oil in the turbomachine maintains a sufficient quality level to provide lubrication, the customer conventionally draws a sample of the oil and sends it to a laboratory for testing. However, some customers improperly draw the oil samples, which can compromise accuracy of the testing. Others do not draw samples frequently enough to properly monitor the condition of the oil.

In other industries, for example, the automotive industry, lubricating oil quality is estimated using empirical data that is tied to an expected lifetime of the oil based upon performance parameters of an automobile. In these cases, an automobile's monitoring system monitors the performance of the vehicle, e.g., speed, acceleration, braking, etc., and based upon the performance of the vehicle, estimates a time at which the lubricating oil will degrade in quality. These automotive systems do not, however, test the lubricating oil to determine its quality.

While more modern conventional systems include particular hardware for sampling and analyzing lubrication oil in turbomachinery, that hardware can be costly to procure and maintain. Further, those hardware-based systems may struggle to accurately assess (e.g., predict) the future quality of lubricating oil in a turbomachine.

BRIEF DESCRIPTION OF THE INVENTION

Various embodiments of the disclosure include a system having: at least one computing device configured to monitor a lubrication oil by performing actions including: determining an initial ideal remaining life for the lubrication oil; determining a temperature-based remaining life for the lubrication oil based upon a temperature measurement of the lubrication oil; calculating a contamination factor of the lubrication oil based upon a non-particle count sample of the lubrication oil; determining an updated ideal life remaining for the lubrication oil based upon the contamination factor, the initial ideal remaining life, and the temperature-based remaining life; and determining an actual life remaining for the lubrication oil based upon the updated ideal life remaining and an actual life lost for the lubrication oil.

Another aspect of the disclosure includes a computer program product including program code, which when executed by one computing device, causes the at least one computing device to monitor a lubrication oil by performing actions including: determining an initial ideal remaining life for the lubrication oil; determining a temperature-based remaining life for the lubrication oil based upon a temperature measurement of the lubrication oil; calculating a contamination factor of the lubrication oil based upon a non-particle count sample of the lubrication oil; determining an updated ideal life remaining for the lubrication oil based upon the contamination factor, the initial ideal remaining life, and the temperature-based remaining life; and determining an actual life remaining for the lubrication oil based upon the updated ideal life remaining and an actual life lost for the lubrication oil.

Another aspect of the disclosure includes a computer-implemented method of monitoring a lubrication oil from a turbomachine, performed on at least one computing device having a processor and a memory, the method including: determining an initial ideal remaining life for the lubrication oil; determining a temperature-based remaining life for the lubrication oil based upon a temperature measurement of the lubrication oil; calculating a contamination factor of the lubrication oil based upon a non-particle count sample of the lubrication oil; determining an updated ideal life remaining for the lubrication oil based upon the contamination factor, the initial ideal remaining life, and the temperature-based remaining life; and determining an actual life remaining for the lubrication oil based upon the updated ideal life remaining and an actual life lost for the lubrication oil.

BRIEF DESCRIPTION OF THE DRAWINGS

These and other features of this disclosure will be more readily understood from the following detailed description of the various aspects of the invention taken in conjunction with the accompanying drawings that depict various embodiments of the disclosure, in which:

FIG. 1 shows a flow diagram illustrating a method performed according to various embodiments of the disclosure.

FIG. 2 shows a graphical depiction of oil lifetime predictions according to ideal estimates, as well as according to various embodiments of the disclosure.

FIG. 3 shows an environment including a system according to various embodiments of the disclosure.

FIG. 4 shows a front schematic view of an apparatus according to various embodiments of the disclosure.

FIG. 5 shows a partial perspective view of the apparatus of FIG. 5 according to embodiments of the disclosure.

It is noted that the drawings of the disclosure are not necessarily to scale. The drawings are intended to depict only typical aspects of the disclosure, and therefore should not be considered as limiting the scope of the disclosure. In the drawings, like numbering represents like elements between the drawings.

DETAILED DESCRIPTION OF THE INVENTION

As indicated above, the subject matter disclosed herein relates to lubrication oil in turbomachinery. More particularly, the subject matter disclosed herein relates to approaches for analyzing a lubricating oil using test data extracted from that oil, exclusive of measured particle-count data.

As noted herein, it can be difficult to effectively monitor the quality of lubricating oil in turbomachine systems, which can lead to undesirable degradation of the oil, and ultimately, damage the turbomachine that relies upon that oil for lubrication.

In contrast to conventional approaches, various embodiments of the disclosure include systems, computer program products and associated methods to analyze a lubricating oil using test data extracted from that oil, exclusive of measured particle-count data. That is, the approaches according to various embodiments of the disclosure are configured to analyze lubricating oil without particle count data measured from that oil. In some particular cases, approaches can include training and deploying a system for analyzing lubricating oil based upon simulation data, measurements of sample oil(s), and mathematical models.

In the following description, reference is made to the accompanying drawings that form a part thereof, and in which is shown by way of illustration specific embodiments in which the present teachings may be practiced. These embodiments are described in sufficient detail to enable those skilled in the art to practice the present teachings and it is to be understood that other embodiments may be utilized and that changes may be made without departing from the scope of the present teachings. The following description is, therefore, merely illustrative.

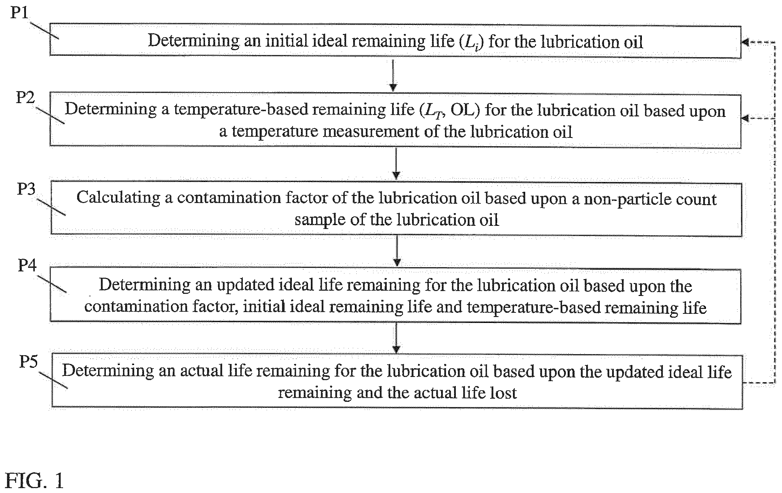

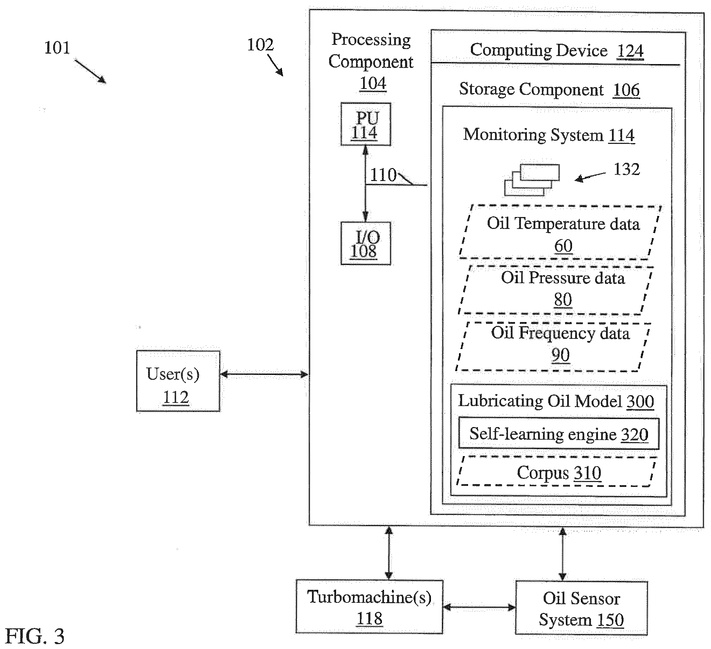

FIG. 1 shows a flow diagram illustrating a process of monitoring a lubrication oil (e.g., a lubrication oil in a turbomachine 118, FIG. 3) according to various embodiments of the disclosure. These processes can be performed, e.g., by at least one computing device, as described herein. For example, these processes may be performed by a lubricating oil monitoring system 114 (or simply, monitoring system 114, FIG. 3). In other cases, these processes can be performed according to a computer-implemented method of monitoring a lubrication oil. In still other embodiments, these processes can be performed by executing computer program code on at least one computing device, causing the at least one computing device to monitor a lubrication oil. In general, the process can include the following subprocesses: Process P1: monitoring system 114 determines an initial ideal remaining life ($L_i$) for the lubrication oil. In various embodiments, this includes obtaining information about the oil type, and calculating the Arrhenius Reaction Rate (ARR) for the oil type, assuming that the oil is clean (free of contaminants), and operates at its design temperature (optimal conditions). The initial ideal remaining life is the amount of life expected of the lubrication oil if it ran under these optimal conditions for its entire life.

The ARR is a known technique used to calculate the oxidation life drop (L) in a mineral oil. The ARR can be calculated according to the following equation in particular embodiments:

$$k = Ae^{-E_a/(RT)} \qquad \text{(Equation 1)}$$

Where k = the rate constant of a chemical reaction; T = absolute temperature of the lubrication oil (in kelvin); A = the pre-exponential factor; $E_a$ = the activation energy of the lubrication oil; and R = the Universal gas constant. Alternatively, the Universal gas constant (R) can be replaced with the Boltzmann constant ($k_B$). Simplified in the case of a mineral oil, the ARR can be represented in terms of an oxidation life (L) of the oil, the rate constant of the chemical reaction ($k_1$), and an ideal rate constant $k_2 = 4750$ as:

$$\mathrm{Log}(L_i) = k_1 + (k_2/\mathrm{T}) \qquad \text{(Equation 2)}$$

Process P2: monitoring system 114 determines a temperature-based remaining life ($L_T$) for the lubrication oil based upon a temperature measurement of the lubrication oil. The temperature-based remaining life can represent an estimated life remaining as predicted based upon the measured temperature of the lubrication oil. This can include obtaining a measurement of the temperature of the lubrication oil. In the case that the lubrication oil is from a turbomachine, the temperature measurement may be obtained from a temperature sensor contacting the lubrication oil, either within the turbomachine, or external to the turbomachine. As with process PI, the temperature-based remaining life can be calculated according to the ARR. However, in some other cases, a temperature-based remaining life (OL), for a given reference temperature To, can be calculated according to the following equation:

$$OL = 10^{\,k_1 + \frac{4750}{T_0}} \qquad \text{(Equation 3)}$$

Where $k_t$ is a constant value attributed to the type of oil being tested, and $T_0$ is a design reference temperature for the oil, in Kelvin. The temperature-based remaining life (OL) describes the life cycle of the lubrication oil at the reference temperature (e.g., temperature at which lubrication oil is designed to operate). The temperature-based remaining life (OL) will be shorter if the oil contains particulates or other contaminates, or if viscosity degrades.

Process P3: monitoring system 114 calculates a contamination factor of the lubrication oil based upon a non-particle count sample of the lubrication oil. In various embodiments, the calculating includes assigning a qualitative weighted contamination factor to each of a plurality of measured oil properties noted herein. In various embodiments, a first oil property A is assigned a weighted contamination factor X, while a second oil property B is assigned a distinct weighted contamination factor of Y x X, where Y is a factor, e.g., 1, 2, 3, 0.1, 0.2, 0.3 a negative factor, percentage factor, etc. In various embodiments, the non-particle count sample can be obtained from a substantially similar sample of the lubrication oil as the temperature measurement, and can include a pressure measurement of the lubrication oil, a frequency measurement of the lubrication oil and/or a temperature-based viscosity calculation of the lubrication oil. In various embodiments, the non-particle count sample is a sample of the lubrication oil that is not analyzed for conventional particle count factors, e.g., a ferrous particle count, water content, dielectric constant, and/or an international organization for standardization (ISO) particle level to calculate a contamination factor. That is, an oil sensor system 150 (FIG. 3) utilized according to various embodiments described herein may not include, or not engage components for analyzing particle count. In some particular cases, contamination factor (TCF) is calculated according to the following equations, based upon a measured temperature, frequency and/or pressure of the lubrication oil:

Viscosity:

$$\mu_{no\min al} = \frac{\mu}{\mu_0} = 8256.9 * T^{-1.822} \qquad \text{(Equation 4)}$$

Where T is the temperature reading of the lubrication oil, e.g., at a temperature sensor, $\mu_{nominal}$ is a normalized lubrication oil viscosity, $\mu$ is a measured actual lubrication oil viscosity, and $\mu_0$ is a reference lubrication oil viscosity.

Pressure:

$$P_{norm} = \frac{P_{pump}}{P_0 \bullet \mu_{no\min ial}} \qquad \text{(Equation 5)}$$

Where $P_{norm}$ is the normalized pressure, $P_0$ is the pump rated pressure at the oil pump, and $P_{pump}$ is the lubrication oil pump pressure, in pounds per square inch (psi).

Total contamination factor (TCF):

$$TCF = k0 + k_2 \bullet P_{norm} + k_3 \bullet P_{norm}^2 + k_4 \bullet \Delta P_{filter} + k_5 \bullet \Delta P_{filter}^2 \ (\text{Equation } 6)$$

Where $\Delta P_{filter}$ is the lubrication oil filter pressure differential, in psid, and $k_0$, $k_2$, $k_3$, $k_4$ and ks are dynamically assigned weights based upon empirical data gathered by oil sensor system(s) 150 (FIG. 3) from machines (e.g., turbomachines 118) operating with lubrication oil and/or models built to simulate operation of lubrication oil from previously gathered empirical data. These weights can be updated based upon conditions to which the lubrication oil is exposed at installation (installation conditions) and/or during operation (operating conditions).

In various embodiments, the total contamination factor (TCF) can be used to determine a lubrication oil life loss factor (LLF), which may be useful in modeling the updated ideal remaining life (ROL) of the lubrication oil. Life loss factor (LLF) can be calculated according to:

$$LLF = TCF * 10^{\frac{4750(T-T_0)}{T \bullet T_0}} \quad (\text{Equation } 7)$$

Where TCF is the total contamination factor (Equation 6), and T is the lubrication oil temperature reading, in Kelvin.

Process P4: monitoring system 114 determines an updated ideal life remaining for the lubrication oil based upon the contamination factor, the initial ideal remaining life, and the temperature-based remaining life. In various embodiments, the updated ideal life remaining for the lubrication oil is calculated by subtracting an actual life (of the lubrication oil) lost from the initial ideal life remaining. In equation form: updated ideal life remaining (ROL) = initial ideal life remaining (OL) - actual life lost (ALL), or:

$$ROL = OL - ALL \ (\text{Equation } 8)$$

The actual life lost can be calculated based upon the total contamination factor (TCF) as a function of time (t) and the measured lubrication oil drain temperature (measured at the drain location on turbomachine 118, FIG. 3) as a function of time (t). In equation form:

$$ALL = \int_0^{t_c} TCF(t) \bullet 10^{\frac{4750(T(t)-T_0)}{T(t)-T_0}} \ dt \ \ (\text{Equation } 9)$$

Where $T_0$ is a reference temperature of the lubrication oil, TCF(t) is the total contamination factor (Equation 6) at a given time (t), and T(t) is the measured lubrication oil drain temperature at time (t).

Process P5: monitoring system 114 determines an actual life remaining for the lubrication oil based upon the updated ideal life remaining and the actual life lost. In various embodiments, the actual life remaining is calculated based upon a difference between the updated ideal life remaining (ROL) and a function of the actual life lost (ALL) over an average time. In equation form:
If:

$$ROL - \int_0^t TCF(t) \bullet 10^{\frac{4750(T_{ave}-T_0)}{T_{ave}-T_0}} \ dt < 0 \ \ \ \ \ \ \ \ \ (\text{Equation } 10)$$

Then: oil requires changing at time (t); where $T_{ave}$ is the average lubrication oil temperature as defined by:

$$T_{ave} = \frac{1}{t_c} \int\limits_{0}^{t_c} T(t)dt \qquad \text{(Equation 11)}$$

In many embodiments, samples of the lubrication oil are obtained at various locations of the turbomachine. In these cases, it is understood that sample data may be averaged or otherwise normalized in order to determine a remaining life.

In some cases, for the first sample data obtained (e.g., temperature data 60, pressure data 80, frequency data 90, etc., FIG. 3), the life loss factor (LLF) can be multiplied by the time between obtaining samples and subtract the value from the life of the fluid under optimal conditions. As noted, this particular example applies to the case of the first sample obtained (or the first sample taken after oil has been changed out of the turbomachine and reservoir). After a first data sample is available, subsequent samples will form part of a running average that factors in some or all of the previously obtained samples.

In particular embodiments, the life loss factor (LLF) can be calculated as a running average based upon a period of operation of the machine including the lubrication oil (e.g., a turbomachine). In some cases, the life loss factor (LLF) is a running average taken over a recent (e.g., most recent) period such as the last 1-3 weeks of operation of the turbomachine.

In various embodiments, Processes P1-P5 can be iterated (repeated) periodically (e.g., according to schedule of x times per y period, and/or continuously) in order to monitor the actual life remaining for a lubrication oil. In some cases, processes P2-P5 can be repeated, for example, by obtaining new sample(s) of the lubrication oil and performing associated processes described herein. In these cases, process P1 may not need to be repeated because the initial ideal life remaining ($L_i$) may be substantially unchanged between some testing intervals.

In various additional embodiments, a model of the lubrication oil can be constructed such that the total contamination factor (TCF) can be modeled based upon trends in lubrication oil data, e.g., temperature reading trends, pressure reading treads, etc. TCF can provide a quantification of particle concentration level and types of particle that cause lube oil to lose its usable service life. According to various embodiments, the TCF can be modeled according to the following:

$$TCF(t) = w_0 + w_1 \bullet t + w_2 \bullet t^2 \qquad \text{(Equation 12)}$$

Where t is a time in the future, and $w_0$, $w_1$, and $w_2$ are weights dynamically updated with trends in lubrication oil data taken from a corpus of data. In various embodiments, for example, as shown and described with respect to the FIG. 3, a lubricating oil model 300 can be constructed using a corpus of data (corpus) 310 about one or more turbomachine(s) 118 and the lubricating oil in those machines. For example, in some cases, a lubricating oil model 300 can be constructed based upon data gathered through processes described herein (e.g., oil temperature data 60, oil pressure data 80, oil frequency data 90), and relationships between that stored data and an expected remaining life of the oil can be calculated. Lubricating oil model 300 may include control logic for analyzing corpus 310 according to one or more mathematical relationships disclosed herein, e.g., Equation 1 through Equation 12. In some cases, corpus 310 can include known relationships between measured characteristics of lubricating oils (e.g., temperature, pressure, frequency, degradation time, etc.) and types of oils, types of turbomachines using those oils, and times at which lubricating oils failed or required maintenance. Lubricating oil model 300 can be continuously or periodically updated with data from corpus 310, which may itself be updated based upon data gathered from one or more turbomachines 118. Lubricating oil model 300 can also include a self-learning engine 320 for deriving new relationships between data from corpus 310 in order to predict a future lubricating oil failure or replacement time, e.g., by continuously updating weights wo, $w_1$, $w_2$ in Equation 12 to dynamically calculate a total contamination factor (TCF) for a given lubricating oil.

It is understood that in the flow diagrams shown and described herein, other processes may be performed while not being shown, and the order of processes can be rearranged according to various embodiments. Additionally, intermediate processes may be performed between one or more described processes. The flow of processes shown and described herein is not to be construed as limiting of the various embodiments.

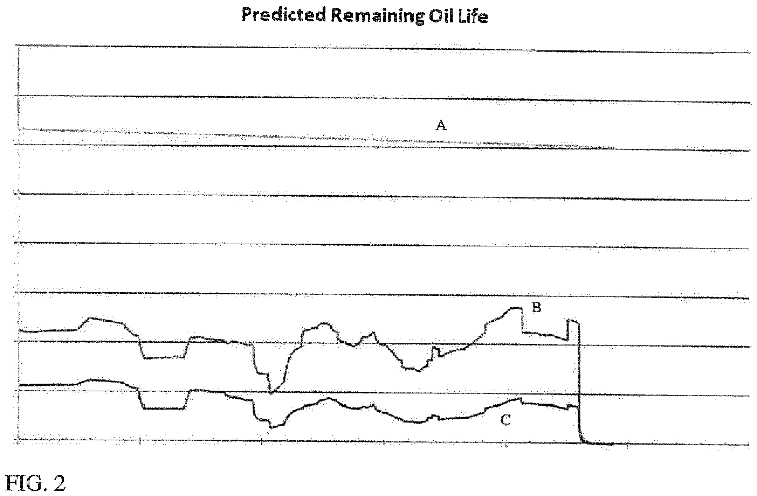

FIG. 2 shows an example graphical depiction of predicted remaining oil life curves according to: A) A theoretical calculation of remaining oil life based upon ideal conditions; B) A predicted life remaining for the oil using approaches according to various embodiments of the disclosure; and C) A predicted lower-limit of remaining life for the oil. Time in years (remaining life) is shown on the Y-axis, and sampling times are shown on the Y-axis.

FIG. 3 shows an illustrative environment 101 including a monitoring system 114, for performing the functions described herein according to various embodiments of the invention. To this extent, the environment 101 includes a computer system 102 that can perform one or more processes described herein in order to monitor a lubrication oil, e.g.,

from one or more turbomachine(s) 118. In particular, the computer system 102 is shown as including the monitoring system 114, which makes computer system 102 operable to monitor a lubrication oil by performing any/all of the processes described herein and implementing any/all of the embodiments described herein. As discussed herein, monitoring system 114 can include a lubricating oil model 300 for predicting failure and/or replacement times for lubricating oils in turbomachine(s) 118.

The computer system 102 is shown including a computing device 124, which can include a processing component 104 (e.g., one or more processors), a storage component 106 (e.g., a storage hierarchy), an input/output (I/O) component 108 (e.g., one or more I/O interfaces and/or devices), and a communications pathway 110. In general, the processing component 104 executes program code, such as the monitoring system 114, which is at least partially fixed in the storage component 106. While executing program code, the processing component 104 can process data, which can result in reading and/or writing transformed data from/to the storage component 106 and/or the I/O component 108 for further processing. The pathway 110 provides a communications link between each of the components in the computer system 102. The I/O component 108 can comprise one or more human I/O devices, which enable a user (e.g., a human and/or computerized user) 112 to interact with the computer system 102 and/or one or more communications devices to enable the system user 112 to communicate with the computer system 102 using any type of communications link. To this extent, the monitoring system 114 can manage a set of interfaces (e.g., graphical user interface(s), application program interface, etc.) that enable human and/or system users 112 to interact with the monitoring system 114. Further, the monitoring system 114 can manage (e.g., store, retrieve, create, manipulate, organize, present, etc.) data, such as oil temperature data 60 (e.g., data about the temperature of the oil, obtained by sensor system 150), oil pressure data 80 (e.g., data about the pressure level of the oil, obtained by sensor system 150) and/or oil frequency data 90 (e.g., data about the frequency measurement of the oil, as obtained by sensor system 150) using any solution. The oil temperature data 60, oil pressure data 80, and/or oil frequency data 90 may be stored as a corpus 310 for later use, in some embodiments. The monitoring system 114 can additionally communicate with a turbomachine 118 and/or an oil sensor system 150 via wireless and/or hardwired means. As noted herein, monitoring system 114 can also include a lubricating oil model 300, which can be constructed using monitoring system 114, and can include a self-learning engine 320 for developing and advancing model 300 as corpus 310 is updated.

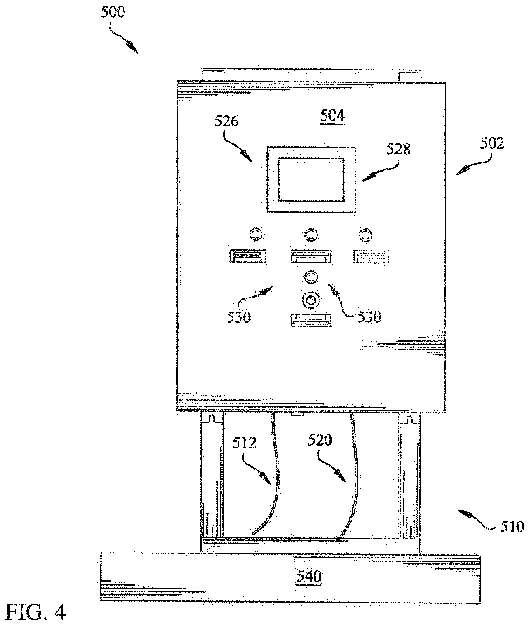

In any event, the computer system 102 can comprise one or more general purpose computing articles of manufacture (e.g., computing devices) capable of executing program code, such as the monitoring system 114, installed thereon. As used herein, it is understood that "program code" means any collection of instructions, in any language, code or notation, that cause a computing device having an information processing capability to perform a particular function either directly or after any combination of the following: (a) conversion to another language, code or notation; (b) reproduction in a different material form; and/or (c) decompression. To this extent, the monitoring system 114 can be embodied as any combination of system software and/or application software. It is further understood that the monitoring system 114 can be implemented in a cloud-based computing environment, where one or more processes are performed at distinct computing devices (e.g., a plurality of computing devices 24), where one or more of those distinct computing devices may contain only some of the components shown and described with respect to the computing device 124 of FIG. 4.

Further, the monitoring system 114 can be implemented using a set of modules 132. In this case, a module 132 can enable the computer system 102 to perform a set of tasks used by the monitoring system 114, and can be separately developed and/or implemented apart from other portions of the monitoring system 114. As used herein, the term "component" means any configuration of hardware, with or without software, which implements the functionality described in conjunction therewith using any solution, while the term "module" means program code that enables the computer system 102 to implement the functionality described in conjunction therewith using any solution. When fixed in a storage component 106 of a computer system 102 that includes a processing component 104, a module is a substantial portion of a component that implements the functionality. Regardless, it is understood that two or more components, modules, and/or systems may share some/all of their respective hardware and/or software. Further, it is understood that some of the functionality discussed herein may not be implemented or additional functionality may be included as part of the computer system 102.

When the computer system 102 comprises multiple computing devices, each computing device may have only a portion of monitoring system 114 fixed thereon (e.g., one or more modules 132). However, it is understood that the computer system 102 and monitoring system 114 are only representative of various possible equivalent computer systems that may perform a process described herein. To this extent, in other embodiments, the functionality provided by the computer system 102 and monitoring system 114 can be at least partially implemented by one or more computing devices that include any combination of general and/or specific purpose hardware with or without program code. In each embodiment, the hardware and program code, if included, can be created using standard engineering and programming techniques, respectively.

Regardless, when the computer system 102 includes multiple computing devices 124, the computing devices can communicate over any type of communications link. Further, while performing a process described herein, the computer system 102 can communicate with one or more other computer systems using any type of communications link. In either case, the communications link can comprise any combination of various types of wired and/or wireless links; comprise any combination of one or more types of networks; and/or utilize any combination of various types of transmission techniques and protocols.

Computer system 102 can obtain or provide data, such as oil temperature data 60, oil pressure data 80 and/or oil frequency data 90 using any solution. The computer system 102 can generate oil temperature data 60, oil pressure data 80 and/or oil frequency data 90, from one or more data stores, receive oil temperature data 60, oil pressure data 80 and/or oil frequency data 90, from another system such as the turbomachine 118, oil sensor system 150 and/or the user 112, send oil temperature data 60, oil pressure data 80 and/or oil frequency data 90 to another system, etc.

While shown and described herein as a method and system for monitoring a lubrication oil, it is understood that aspects of the invention further provide various alternative embodiments. For example, in one embodiment, the invention provides a computer program fixed in at least one computer-readable medium, which when executed, enables a computer system to monitor a lubrication oil. To this extent, the computer-readable medium includes program code, such as the monitoring system 114 (FIG. 3), which implements some or all of the processes and/or embodiments described herein. It is understood that the term "computer-readable medium" comprises one or more of any type of tangible medium of expression, now known or later developed, from which a copy of the program code can be perceived, reproduced, or otherwise communicated by a computing device. For example, the computer-readable medium can comprise: one or more portable storage articles of manufacture; one or more memory/storage components of a computing device; paper; etc.

In another embodiment, the invention provides a method of providing a copy of program code, such as the monitoring system 114 (FIG. 3), which implements some or all of a process described herein. In this case, a computer system can process a copy of program code that implements some or all of a process described herein to generate and transmit, for reception at a second, distinct location, a set of data signals that has one or more of its characteristics set and/or changed in such a manner as to encode a copy of the program code in the set of data signals. Similarly, an embodiment of the invention provides a method of acquiring a copy of program code that implements some or all of a process described herein, which includes a computer system receiving the set of data signals described herein, and translating the set of data signals into a copy of the computer program fixed in at least one computer-readable medium. In either case, the set of data signals can be transmitted/received using any type of communications link.

In still another embodiment, the invention provides a method of monitoring a lubrication oil. In this case, a computer system, such as the computer system 102 (FIG. 3), can be obtained (e.g., created, maintained, made available, etc.) and one or more components for performing a process described herein can be obtained (e.g., created, purchased, used, modified, etc.) and deployed to the computer system. To this extent, the deployment can comprise one or more of: (1) installing program code on a computing device; (2) adding one or more computing and/or I/O devices to the computer system; (3) incorporating and/or modifying the computer system to enable it to perform a process described herein; etc.

In any case, the technical effect of the various embodiments of the invention, including, e.g., the monitoring system 114, is to monitor a lubrication oil, e.g., a lubrication oil from a turbomachine (e.g., turbomachine 118). It is understood that the monitoring system 114 could be implemented monitor a lubrication oil in a plurality of distinct applications, e.g., to monitor lubrication oil in an automobile system, to monitor lubrication oil in a piece of heavy machinery, etc.

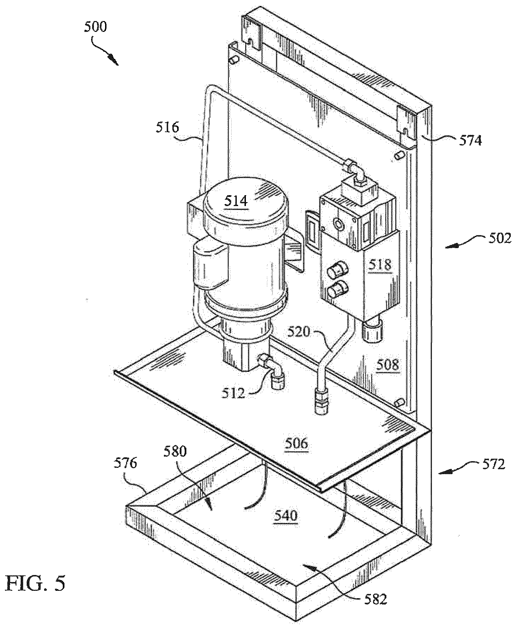

Various additional embodiments can include a lubricating oil monitoring apparatus 500 (FIGS. 4 and 5), which can include one or more components of monitoring system 114 (and associated functionality), along with oil sensor system 150 (FIG. 3). Lubricating oil monitoring apparatus 500 can be configured to non-invasively monitor one or more condition(s) of the lubricating oil. In some cases, lubricating oil monitoring apparatus 500 (and in particular, the oil sensor system 150) can monitor one or more parameters of the lubricating oil, including but not limited to: a temperature of lubricating oil, a pressure of lubricating oil, and a frequency of lubricating oil.

In various embodiments, lubricating oil monitoring apparatus 500 can continuously monitor these parameters, and compare these parameters with acceptable thresholds (e.g., levels or ranges) to determine whether the lubricating oil is at a desired level. Lubricating oil monitoring apparatus 500 can include an interface, e.g., a human-machine interface (HMI) for providing one or more alerts when the determined parameter(s) of the lubricating oil deviate, approach, and/or trend toward an unacceptable threshold/range.

In some cases, lubricating oil monitoring apparatus 500 can be mounted or otherwise coupled with the turbomachine. In other cases, lubricating oil monitoring apparatus 500 is located proximate the turbomachine to provide real-time monitoring of the condition of the lubricating oil.

In various embodiments, lubricating oil monitoring apparatus 500 can be fluidly connected with the existing lubricating oil reservoir in the turbomachine. In some particular embodiments, lubricating oil monitoring apparatus 500 is fluidly connected with the return line drain section of the oil reservoir. In some cases, lubricating oil monitoring apparatus 500 includes an oil supply line for extracting oil from the reservoir, and a drain line for draining tested oil back to the reservoir. Apparatus 500 can also include a mount for mounting onto the reservoir or a proximate portion of the turbomachine.

FIGS. 4 and 5 show a schematic front view and partial perspective view, respectively, of an embodiment of a lubricating oil monitoring apparatus (apparatus) 500 according to various embodiments of the invention. FIG. 4 shows apparatus 500 including a housing section 502 having a casing 504 over a base plate 506 and back support 508 (FIG. 5). FIG. 4 also illustrates a mount 510 coupled with housing section 502. FIG. 5 shows apparatus 500 in perspective view without casing 504, and illustrates oil intake conduit 512, oil pump 514, internal conduit 516, oil analyzer 518, and drain conduit 520. Various components described with respect to apparatus 500 can be formed of conventional materials known in the art, e.g., metals such as steel, copper, aluminum, alloys, composites, etc.

With reference to both FIGS. 4 and 5, in some particular embodiments, lubricating oil monitoring apparatus (apparatus) 500 can include:

A housing section 502 including a base plate 506 and back support 508, which may be formed of a sheet metal or other suitable composite. Housing section 502 can also include a casing 504 coupled to base plate 506 and back support 508, as shown in FIG. 4. In various embodiments, casing 504 can include an interface 526, e.g., a human-machine interface (HMI), which can include a display 528 (e.g., a touch-screen, digital or other display). In some cases, interface 526 can include one or more alert indicator(s) 530, which can include one or more lights (e.g., LEDs), audio indicators and/or tactile indicators for indicating that a condition of the tested oil is approaching, has approached or could approach an undesirable level (e.g., range).

Housing section 502 can also include an oil intake conduit 512 connected with base plate 506 and extending through base plate 506. Oil intake conduit 512 can be fluidly connected with turbomachine oil reservoir (reservoir) 540, and is configured to extract oil from reservoir 540. Also shown (in FIG. 5), housing section 502 can include an oil pump 514 substantially contained within casing 504 and fluidly connected with oil intake conduit 512. Pump 514 can provide pumping pressure to draw the oil from reservoir 540 through oil intake conduit 512 (and above base plate 506). Housing section 502 can further include an internal conduit 516 fluidly connected with oil pump 514 (at an outlet of pump 514) and intake conduit 512. Internal conduit 516 is configured for receiving intake oil from pump 514. Housing section 502 can also include an oil analyzer 518 fluidly connected with internal conduit 516, where oil analyzer 518 measures a characteristic of the intake oil (e.g., an oil temperature, an oil pressure and/or an oil frequency). Also shown, housing section 502 can include a drain conduit 520 fluidly connected with oil analyzer 518, extending through base plate 506, and fluidly connected with reservoir 540. Drain conduit 520 allows for draining of tested oil back to reservoir 540.

Apparatus 500 can also include a mount 510 coupled to housing section 502. Mount 510 can be designed to couple to oil reservoir 540 of a turbomachine.

In various embodiments, base plate 506 is configured to face vertically downward, e.g., run perpendicular to the vertical axis (y). This can allow drain conduit 560 to utilize gravitational forces to drain the tested lubricating oil back to reservoir 540. In these cases, base plate 506 overlies reservoir 540.

In some particular embodiments, mount 510 includes an L-shaped member 572 including a vertically extending spine 574 coupled with housing section 502 and a horizontally extending base 576. Horizontally extending base 576 can be mountable on oil reservoir 540 of the turbomachine 118.

It is understood that apparatus 500 can be powered by a power unit, e.g., a battery power unit, and/or a direct alternating-current (AC) connection with one or more power sources of the turbomachine.

During operation apparatus 500 is configured to extract reservoir oil from oil reservoir 540 via intake conduit 512 (with pump 514 providing the pressure to draw the reservoir oil vertically upward), pump that extracted oil through internal conduit 516, and provide the oil to analyzer 518 for testing prior to releasing the oil back to reservoir 540 via drain conduit 520. In various embodiments, drain conduit 520 empties to a distinct section 580 of reservoir 540 than section 582 coupled with the intake conduit 512. In some cases, reservoir 540 has a substantially continuous flow path going from extraction location 582 toward drain location 580, meaning that new oil is continuously entering reservoir 540 from turbomachine 118, passing through reservoir 540 (and being tested by apparatus 500), and re-entering the turbomachine.

In various embodiments, components described as being "coupled" to one another can be joined along one or more interfaces. In some embodiments, these interfaces can include junctions between distinct components, and in other cases, these interfaces can include a solidly and/or integrally formed interconnection. That is, in some cases, components that are "coupled" to one another can be simultaneously formed to define a single continuous member. However, in other embodiments, these coupled components can be formed as separate members and be subsequently joined through known processes (e.g., fastening, ultrasonic welding, bonding).

This written description uses examples to disclose the invention, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

Various aspects and embodiments of the present invention are defined by the following numbered clauses:

1. A system comprising:
at least one computing device configured to monitor a lubrication oil by performing actions including:

determining an initial ideal remaining life for the lubrication oil;
determining a temperature-based remaining life for the lubrication oil based upon a temperature measurement of the lubrication oil;
calculating a contamination factor of the lubrication oil based upon a non-particle count sample of the lubrication oil;
determining an updated ideal life remaining for the lubrication oil based upon the contamination factor, the initial ideal remaining life, and the temperature-based remaining life; and
determining an actual life remaining for the lubrication oil based upon the updated ideal life remaining and an actual life lost for the lubrication oil.

2. The system of clause 1, wherein the actual life lost is a function of a life loss factor (LLF) for the lubrication oil, the LLF calculated according to:

$$LLF = TCF * 10^{\frac{4750(T-T_0)}{T \bullet T_0}} \quad ,$$

where TCF is the calculated contamination factor T is the temperature measurement of the lubrication oil and $T_0$ is a reference temperature for the lubrication oil.

3. The system of any preceding clause, wherein the at least one computing device is further configured to determine an elapsed time between samplings of the lubrication oil based upon a sample frequency of the lubrication oil.

4. The system of any preceding clause, wherein the determining of the actual life remaining includes determining the actual life lost based upon the contamination factor as a function of time and the temperature measurement of the lubrication oil as a function of time.

5. The system of any preceding clause, wherein the determining of the updated ideal life remaining for the lubrication oil includes calculating the updated ideal life remaining according to:

$$\text{updated ideal life remaining} = \text{initial ideal life remaining} - \text{actual life lost.}$$

6. The system of any preceding clause, wherein the determining of the actual life remaining for the lubrication oil includes calculating the actual life remaining according to:

$$\text{If: } ROL - \int_0^t TCF(t) \bullet 10^{\frac{4750(T_{ave}-T_0)}{T_{ave}-T_0}} dt < 0 ; \qquad \text{Then:}$$

change lubricating oil at time (t),
wherein $T_{ave}$ is an average lubrication oil temperature measured over time, TCF is the contamination factor and $T_0$ is a reference temperature for the lubrication oil.

7. The system of any preceding clause, wherein $T_{ave}$ is calculated according to:

$$T_{ave} = \frac{1}{t_c} \int_0^{t_c} T(t)dt \ .$$

8. The system of any preceding clause, wherein the temperature-based remaining life for the lubrication oil is calculated based upon an Arrhenius Reaction Rate of the lubrication oil.

9. The system of any preceding clause, wherein the contamination factor is calculated according to:

$$TCF = k0 + k_2 \bullet P_{norm} + k_3 \bullet P_{norm}^2 + k_4 \bullet \Delta P_{filter} + k_5 \bullet \Delta P_{filter}^2 \text{ ,}$$

wherein $\Delta P_{filter}$ is a measured lubrication oil filter pressure differential, and $k_0$, $k_2$, $k_3$, $k_4$ and $k_5$ are dynamically assigned weights based upon installation conditions for the lubrication oil or operating conditions for the lubrication oil.

10. A computer program product comprising program code, which when executed by one computing device, causes the at least one computing device to monitor a lubrication oil by performing actions including:

determining an initial ideal remaining life for the lubrication oil;
determining a temperature-based remaining life for the lubrication oil based upon a temperature measurement of the lubrication oil;
calculating a contamination factor of the lubrication oil based upon a non-particle count sample of the lubrication oil;
determining an updated ideal life remaining for the lubrication oil based upon the contamination factor, the initial ideal remaining life, and the temperature-based remaining life; and
determining an actual life remaining for the lubrication oil based upon the updated ideal life remaining and an actual life lost for the lubrication oil.

11. The computer program product of any preceding clause, wherein the actual life lost is a function of a life loss factor (LLF) for the lubrication oil, the LLF calculated according to:

$$LLF = TCF * 10^{\frac{4750(T-T_0)}{T \bullet T_0}} \text{ ,}$$

where TCF is the calculated contamination factor and T is the temperature measurement of the lubrication oil and $T_0$ is a reference temperature for the lubrication oil.

12. The computer program product of any preceding clause, wherein the program code causes the at least one computing device to determine an elapsed time between samplings of the lubrication oil based upon a sample frequency of the lubrication oil.

13. The computer program product of any preceding clause, wherein the determining of the actual life remaining includes determining the actual life lost based upon the contamination factor as a function of time and the temperature measurement of the lubrication oil as a function of time.

14. The computer program product of any preceding clause, wherein the determining of the updated ideal life remaining for the lubrication oil includes calculating the updated ideal life remaining according to:

$$\text{updated ideal life remaining} = \text{initial ideal life remaining} - \text{actual life lost.}$$

15. The computer program product of any preceding clause, wherein the determining of the actual life remaining for the lubrication oil includes calculating the actual life remaining according to:

$$\text{If: } ROL - \int_0^t TCF(t) \bullet 10^{\frac{4750(T_{ave}-T_0)}{T_{ave}-T_0}} \, dt < 0;$$

Then: change lubricating oil at time (t),
wherein $T_{ave}$ is an average lubrication oil temperature measured over time, TCF is the contamination factor and $T_0$ is a reference temperature for the lubrication oil.

16. The computer program of any preceding clause claim 10, wherein the contamination factor is calculated according to:

$$TCF = k0 + k_2 \bullet P_{norm} + k_3 \bullet P_{norm}^2 + k_4 \bullet \Delta P_{filter} + k_5 \bullet \Delta P_{filter}^2 \text{,}$$

wherein $\Delta P_{filter}$ is a measured lubrication oil filter pressure differential, and $k_0$, $k_2$, $k_3$, $k_4$ and $k_5$ are dynamically assigned weights based upon installation conditions for the lubrication oil or operating conditions for the lubrication oil.

17. A computer-implemented method of monitoring a lubrication oil from a turbomachine, performed on at least one computing device having a processor and a memory, the method comprising:

determining an initial ideal remaining life for the lubrication oil;
determining a temperature-based remaining life for the lubrication oil based upon a temperature measurement of the lubrication oil;
calculating a contamination factor of the lubrication oil based upon a non-particle count sample of the lubrication oil;
determining an updated ideal life remaining for the lubrication oil based upon the contamination factor, the initial ideal remaining life, and the temperature-based remaining life; and
determining an actual life remaining for the lubrication oil based upon the updated ideal life remaining and an actual life lost for the lubrication oil.

18. The computer-implemented method of any preceding clause, wherein the determining of the actual life remaining includes determining the actual life lost based upon the contamination factor as a function of time and the temperature measurement of the lubrication oil as a function of time.

19. The computer-implemented method of any preceding clause, wherein the determining of the actual life remaining for the lubrication oil includes calculating the actual life remaining according to:

$$\text{If: } ROL - \int_0^t TCF(t) \bullet 10^{\frac{4750(T_{ave}-T_0)}{T_{ave}-T_0}} \, dt < 0;$$

Then: change lubricating oil at time (t),

wherein $T_{ave}$ is an average lubrication oil temperature measured over time, TCF is the contamination factor and $T_0$ is a reference temperature for the lubrication oil.

20. The computer-implemented method of any preceding clause, wherein the contamination factor is calculated according to:

$$TCF = k0 + k_2 \bullet P_{norm} + k_3 \bullet P_{norm}^2 + k_4 \bullet \Delta P_{filter} + k_5 \bullet \Delta P_{filter}^2 \text{,}$$

wherein $\Delta P_{filter}$ is a measured lubrication oil filter pressure differential, and $k_0$, $k_2$, $k_3$, $k_4$ and $k_5$ are dynamically assigned weights based upon installation conditions for the lubrication oil or operating conditions for the lubrication oil.

**Claims**

1.  A system (102) comprising:
    at least one computing device (124) configured to monitor a lubrication oil (300) by performing actions including:

determining an initial ideal remaining life for the lubrication oil (300);
determining a temperature-based remaining life for the lubrication oil (300) based upon a temperature measurement of the lubrication oil (300);
calculating a contamination factor of the lubrication oil (300) based upon a non-particle count sample of the lubrication oil (300);
determining an updated ideal life remaining for the lubrication oil (300) based upon the contamination factor, the initial ideal remaining life, and the temperature-based remaining life; and
determining an actual life remaining for the lubrication oil (300) based upon the updated ideal life remaining and an actual life lost for the lubrication oil (300).

2. The system (102) of claim 1, wherein the actual life lost is a function of a life loss factor (LLF) for the lubrication oil (300), the LLF calculated according to:

$$LLF = TCF * 10^{\frac{4750(T-T_0)}{T \bullet T_0}} \quad ,$$

where TCF is the calculated contamination factor T is the temperature measurement of the lubrication oil (300) and $T_0$ is a reference temperature for the lubrication oil (300).

3. The system (102) of claim 1 or 2, wherein the at least one computing device (124) is further configured to determine an elapsed time between samplings of the lubrication oil (300) based upon a sample frequency of the lubrication oil (300).

4. The system (102) of claim 1, 2 or 3, wherein the determining of the actual life remaining includes determining the actual life lost based upon the contamination factor as a function of time and the temperature measurement of the lubrication oil (300) as a function of time.

5. The system (102) of claim 4, wherein the determining of the updated ideal life remaining for the lubrication oil (300) includes calculating the updated ideal life remaining according to:

$$\text{updated ideal life remaining} = \text{initial ideal life remaining} - \text{actual life lost.}$$

6. The system (102) of claim 1, 2 or 3, wherein the determining of the actual life remaining for the lubrication oil (300) includes calculating the actual life remaining according to:

$$\text{If: } ROL - \int_0^t TCF(t) \bullet 10^{\frac{4750(T_{ave}-T_0)}{T_{ave}-T_0}} \, dt < 0; \quad \text{Then:}$$

change lubricating oil (300) at time (t),
wherein $T_{ave}$ is an average lubrication oil (300) temperature measured over time, TCF is the contamination factor and $T_0$ is a reference temperature for the lubrication oil (300).

7. The system (102) of claim 6, wherein $T_{ave}$ is calculated according to:

$$T_{ave} = \frac{1}{t_c} \int_0^{t_c} T(t) dt \; .$$

8. The system (102) of any preceding claim, wherein the temperature-based remaining life for the lubrication oil (300) is calculated based upon an Arrhenius Reaction Rate of the lubrication oil (300).

9. The system (102) of any preceding claim, wherein the contamination factor is calculated according to:

$$TCF = k0 + k_2 \bullet P_{norm} + k_3 \bullet P_{norm}^2 + k_4 \bullet \Delta P_{filter} + k_5 \bullet \Delta P_{filter}^2 ,$$

wherein $\Delta P_{filter}$ is a measured lubrication oil filter pressure differential, and $k_0$, $k_2$, $k_3$, $k_4$ and ks are dynamically assigned weights based upon installation conditions for the lubrication oil (300) or operating conditions for the lubrication oil (300).

10. A computer-implemented method of monitoring a lubrication oil (300) from a turbomachine (118), performed on at least one computing device (124) having a processor (104) and a memory (106), the method comprising:

determining an initial ideal remaining life for the lubrication oil (300);

determining a temperature-based remaining life for the lubrication oil (300) based upon a temperature measurement of the lubrication oil (300);

calculating a contamination factor of the lubrication oil (300) based upon a non-particle count sample of the lubrication oil (300);

determining an updated ideal life remaining for the lubrication oil (300) based upon the contamination factor, the initial ideal remaining life, and the temperature-based remaining life; and

determining an actual life remaining for the lubrication oil (300) based upon the updated ideal life remaining and an actual life lost for the lubrication oil (300).

P1
Determining an initial ideal remaining life ($L_i$) for the lubrication oil

P2
Determining a temperature-based remaining life ($L_T$, OL) for the lubrication oil based upon a temperature measurement of the lubrication oil

P3
Calculating a contamination factor of the lubrication oil based upon a non-particle count sample of the lubrication oil

P4
Determining an updated ideal life remaining for the lubrication oil based upon the contamination factor, initial ideal remaining life and temperature-based remaining life

P5
Determining an actual life remaining for the lubrication oil based upon the updated ideal life remaining and the actual life lost

FIG. 1

**Predicted Remaining Oil Life**

FIG. 2

101

102

**Processing Component 104**

PU 114

110

I/O 108

User(s) 112

**Computing Device 124**

Storage Component 106

Monitoring System 114

132

Oil Temperature data 60

Oil Pressure data 80

Oil Frequency data 90

Lubricating Oil Model 300

Self-learning engine 320

Corpus 310

Turbomachine(s) 118

Oil Sensor System 150

FIG. 3

FIG. 4

FIG. 5

Europäisches
Patentamt

European
Patent Office

Office européen
des brevets

# EUROPEAN SEARCH REPORT

Application Number

EP 18 17 3490

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/324361 A1 (O'DONNELL KEEGAN SAUNDERS [US] ET AL) 30 October 2014 (2014-10-30) * paragraphs [0002], [0007], [0027] - [0031]; claims 1-7; figure 1 * | 1-10 | INV. F01M11/10 F02C9/00 |
| A | US 2017/081997 A1 (POTYRAILO RADISLAV ALEXANDROVICH [US] ET AL) 23 March 2017 (2017-03-23) * paragraphs [0012], [0148], [0181] * | 1-10 | |
| A | US 7 581 434 B1 (DISCENZO FREDERICK M [US] ET AL) 1 September 2009 (2009-09-01) * abstract * * column 24, lines 28-35; table 6 * * column 35, lines 30-67 * * column 36, lines 1-17 * | 1-10 | |
| A | EP 2 995 788 A1 (ROLLS ROYCE CORP [US]; ROLLS ROYCE NAM TECH INC [US]) 16 March 2016 (2016-03-16) * paragraphs [0015] - [0016] * | 1-10 | TECHNICAL FIELDS SEARCHED (IPC) |
| A | EP 2 085 776 A1 (HONEYWELL INT INC [US]) 5 August 2009 (2009-08-05) * paragraphs [0002], [0006] * | 1-10 | F01M F02C F01D F16N |
| A | US 2007/074562 A1 (LIU JAMES Z [US] ET AL) 5 April 2007 (2007-04-05) * paragraphs [0023] - [0024] * | 1-10 | |
| A | US 2003/222656 A1 (PHILLIPS ALAN D [US] ET AL) 4 December 2003 (2003-12-04) * paragraphs [0006], [0022], [0035], [0148] * | 1-10 | |
| A | US 2014/007657 A1 (MATSUBARA YUKIO [JP] ET AL) 9 January 2014 (2014-01-09) * paragraphs [0023], [0025] - [0026] * | 1-10 | |

The present search report has been drawn up for all claims

| Place of search | Date of completion of the search | Examiner |
|---|---|---|
| The Hague | 5 October 2018 | Ducloyer, Stéphane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 406 867 A1**

## ANNEX TO THE EUROPEAN SEARCH REPORT
## ON EUROPEAN PATENT APPLICATION NO.

EP 18 17 3490

This annex lists the patent family members relating to the patent documents cited in the above-mentioned European search report.
The members are as contained in the European Patent Office EDP file on
The European Patent Office is in no way liable for these particulars which are merely given for the purpose of information.

05-10-2018

| Patent document cited in search report | | Publication date | Patent family member(s) | | Publication date |
|---|---|---|---|---|---|
| US 2014324361 | A1 | 30-10-2014 | CH | 708012 A2 | 31-10-2014 |
| | | | CN | 104141515 A | 12-11-2014 |
| | | | DE | 102014105571 A1 | 30-10-2014 |
| | | | JP | 2014214875 A | 17-11-2014 |
| | | | US | 2014324361 A1 | 30-10-2014 |
| US 2017081997 | A1 | 23-03-2017 | NONE | | |
| US 7581434 | B1 | 01-09-2009 | NONE | | |
| EP 2995788 | A1 | 16-03-2016 | EP | 2995788 A1 | 16-03-2016 |
| | | | US | 2016076491 A1 | 17-03-2016 |
| EP 2085776 | A1 | 05-08-2009 | EP | 2085776 A1 | 05-08-2009 |
| | | | US | 2009192728 A1 | 30-07-2009 |
| US 2007074562 | A1 | 05-04-2007 | US | 2007074562 A1 | 05-04-2007 |
| | | | WO | 2007044308 A1 | 19-04-2007 |
| US 2003222656 | A1 | 04-12-2003 | AU | 2002366698 A1 | 09-07-2003 |
| | | | EP | 1474676 A2 | 10-11-2004 |
| | | | US | 2003222656 A1 | 04-12-2003 |
| | | | WO | 03054482 A2 | 03-07-2003 |
| US 2014007657 | A1 | 09-01-2014 | CN | 103460009 A | 18-12-2013 |
| | | | EP | 2682732 A1 | 08-01-2014 |
| | | | ES | 2657592 T3 | 06-03-2018 |
| | | | US | 2014007657 A1 | 09-01-2014 |
| | | | WO | 2012117970 A1 | 07-09-2012 |

EPO FORM P0459

For more details about this annex : see Official Journal of the European Patent Office, No. 12/82

21